(19) Europäisches Patentamt
European Patent Office
Office européen des brevets

(11) Veröffentlichungsnummer: **0 042 059**
**B1**

(12) # EUROPÄISCHE PATENTSCHRIFT

(45) Veröffentlichungstag der Patentschrift: **31.08.83**

(51) Int. Cl.³: **C 07 C 118/04,** C 07 C 119/042, C 07 C 119/045, C 07 C 119/048

(21) Anmeldenummer: **81103577.3**

(22) Anmeldetag: **11.05.81**

(54) **Verfahren zur Herstellung von Isocyanaten.**

(30) Priorität: **06.06.80 DE 3021299**

(43) Veröffentlichungstag der Anmeldung: **23.12.81 Patentblatt 81/51**

(45) Bekanntmachung des Hinweises auf die Patenterteilung: **31.08.83 Patentblatt 83/35**

(84) Benannte Vertragsstaaten: **BE CH DE FR GB LI**

(56) Entgegenhaltungen:
**DE-B-1 944 719**
**FR-A-2 230 624**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Towae, Friedrich, Dr., Parkstrasse 22, D-6700 Ludwigshafen (DE)**

**EP 0 042 059 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Isocyanaten

Die Erfindung betrifft ein Verfahren zur Herstellung von Isocyanaten durch thermische Zersetzung von Oxalsäureesteramiden bei einer Temperatur von 300 bis 900°C.

Isocyanate werden im allgemeinen durch Phosgenierung von primären Aminen hergestellt (Houben-Weyl, Methoden der Organischen Chemie, Band VIII, Seiten 119 bis 121), jedoch besitzt dieses Verfahren wegen der Toxizität des Phosgens sowie der Korrosivität des HCl und des damit verbundenen hohen technologischen Aufwands schwerwiegende Nachteile. So entstehen hohe Investitionskosten, speziell bei der Herstellung geringer Mengen an Isocyanaten.

Es ist bekannt, Isocyanate aus Carbamidsäurechloriden in Gegenwart von organischen Basen wie tertiären Aminen oder N,N-Dialkylcarbonsäureamiden (DE-OS 15 93 554) in organischen Lösungsmitteln herzustellen. Auch mit wässrigen Lösungen oder Suspensionen von anorganischen Basen, wie Alkalihydroxiden, Erdalkalihyroxiden, Alkalicarbonaten, Erdalkalicarbonaten oder Alkalihydrogencarbonaten können Isocyanate erhalten werden (GB-PS 1 208 862). US-Patentschrift 3 465 023 weist ausdrücklich darauf hin, dass die Bildung von Chlorwasserstoff bei der Isocyanatherstellung die Reaktionsfreudigkeit der Endstoffe herabsetzt und daher die Bindung der Säure bei dem Verfahren wichtig ist. Auch ergeben sich Schwierigkeiten bei der Destillation des Isocyanats und Korrosion in den Anlagen. Die genannten Verfahren haben den Nachteil, dass die Isocyanate in einem Medium entstehen, in dem sie gegen Zersetzung empfindlich sind. So ist es aus Houben-Weyl, Methoden der Organischen Chemie, Band VIII, Seite 136 (1952) bekannt, dass Isocyanate in Gegenwart tertiärer Amine dimerisieren. Gegenüber wässrigem Alkali sind sie äusserst instabil und gehen selbst bei Verwendung stöchiometrischer Mengen an wässrigem Alkali zu einem grossen Teil in Carbaminate bzw. Carbamidsäuren über.

Es ist aus der deutschen Patentschrift 1 193 034 bekannt, dass man N-Alkylcarbamidsäurechloride mit Alkylgruppen von 1 bis 3 Kohlenstoffatomen in einem organischen Lösungsmittel zersetzt, wobei der gebildete Chlorwasserstoff über einen Rückflusskühler und gleichzeitig das gebildete Alkylisocyanat durch Destillation über eine Kolonne aus dem Reaktionsraum entfernt werden. Das Lösungsmittel muss einen Siedepunkt von mindestens 10°C über dem Siedepunkt des gebildeten Alkylisocyanats besitzen. Es wird darauf hingewiesen, dass sich Isocyanate, deren Siedepunkt unter dem Zersetzungspunkt der entsprechenden Carbamidsäurechloride liegen, nicht durch Kochen der Carbamidsäurechloride unter Rückfluss herstellen lassen; solche Isocyanate sind z.B. Alkylisocyanate. Die Patentschrift lehrt, dass in solchen Fällen zwar die Carbamidsäurechloride thermisch gespalten werden, sich aber ein Gleichgewicht einstellt und sich ein grosser Teil des Chlorwasserstoffs wieder mit dem gebildeten Isocyanat zum Ausgangsstoff zurückbildet.

In jüngerer Zeit wurde versucht, kostengünstigere und problemlosere Verfahren zur Isocyanatsynthese zu entwickeln. So wurde beispielsweise vorgeschlagen, aromatische Isocyanate direkt aus den entsprechenden Nitroverbindungen und Kohlenmonoxid unter dem katalytischen Einfluss von Edelmetallen herzustellen (DE-PS 1 815 517, DE-PS 1 909 190, DE-PS 1 944 747). Dieses Verfahren ist jedoch nur für aromatische Isocyanate brauchbar und erfordert wegen des Einsatzes der Edelmetallkatalysatoren einen erheblichen Kostenaufwand.

Ein weiterer Weg zu Isocyanaten besteht in der thermischen Spaltung von Urethanen (DE-OS 26 35 490, DE-OS 24 10 505), die aus Nitroverbindungen, Kohlenmonoxid und Alkohol erhalten werden (DE-OS 26 23 694, EP-Anm. 0 000 563, DE-OS 26 14 101). Dieses Verfahren wurde bislang ebenfalls nur für aromatische Isocyanate angewandt und ist zudem auch aufwendig, da die Herstellung der Urethanvorprodukte hohe Drükke und die Verwendung von Katalysatoren benötigt, die entweder sehr teuer sind, z.B. Edelmetalle, oder die toxische Nebenprodukte bilden können, z.B. Selenverbindungen. Es wurde auch vorgeschlagen, Urethane durch Umsetzung von Diaryl-, Dialkylaryl- oder Dialkylcarbonaten mit Aminen herzustellen (DE-OS 21 60 111). Diese Verfahrensvariante erlaubt jedoch die Bildung aromatischer Urethane nur mit mässigen Raumzeitausbeuten, wobei ausserdem noch eine sorgfältige Reaktionsführung nötig ist, da sonst erhebliche Mengen alkylierter Amine als Nebenprodukte entstehen. Alle diese Verfahren sind so im Hinblick auf wirtschaftlichen und einfachen Betrieb, geringen apparativen Aufwand und leicht zugängliche Ausgangsstoffe unbefriedigend.

Es wurde nun gefunden, dass man Isocyanate der Formel

$R^1-N=C=O$ I,

worin $R^1$ einen aliphatischen, cycloaliphatischen, araliphatischen, aliphatisch-aromatischen oder aromatischen Rest bezeichnet, durch thermische Spaltung von Carbonamidoverbindungen vorteilhaft erhält, wenn man Oxalsäureesteramide der Formel

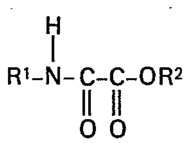 II,

worin $R^1$ die vorgenannte Bedeutung besitzt und $R^2$ einen aliphatischen, cycloaliphatischen, araliphatischen, aliphatisch-aromatischen oder aromatischen Rest bezeichnet, bei einer Temperatur von 300 bis 900°C thermisch spaltet.

Die Umsetzung kann für den Fall der Verwendung von Oxalsäuremethylester-N-phenylamid durch die folgenden Formeln wiedergegeben werden:

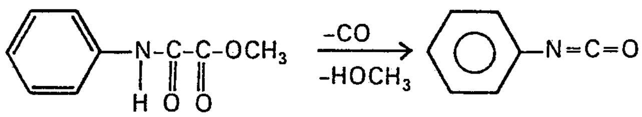


Im Vergleich zu den bekannten Verfahren liefert das Verfahren nach der Erfindung auf einfacherem und wirtschaftlicherem Wege Isocyanate in sehr guter Ausbeute und Reinheit. Im Vergleich zu dem in der deutschen Patentschrift 1 193 034 beschriebenen Verfahren ist das Verfahren nach der Erfindung betriebssicherer. Die Abspaltung und Entfernung des giftigen Halogenwasserstoffs werden vermieden und die Betriebssicherheit und der Schutz des Betriebspersonals somit wesentlich gefördert. Die von der US-Patentschrift erwähnten Schwierigkeiten in bezug auf Destillation, Korrosionsprobleme und Herabsetzung der Reaktivität der Endstoffe treten nicht in wesentlichem Masse auf. Teure Lösungsmittel und zusätzliche Basen werden nicht benötigt. Diese vorteilhaften Ergebnisse sind überraschend, denn nach dem Stand der Technik war insbesondere Bildung von Polymeren bzw. Zersetzungsprodukten unter den erfindungsgemässen hohen Temperaturen zu erwarten. Im Hinblick auf die vorgenannten, Urethane bzw. Nitroverbindungen als Ausgangsstoffe verwendeden Verfahren, sind die erfindungsgemässen Ausgangsstoffe Amine und Oxalester leichter zugänglich. Auch ist vorteilhaft, die toxischen und korrosiven Chlorkohlensäureester, eine teure Selen- oder Edelmetall katalysierte reduktive Carbonylierung von Nitroverbindungen mit ihren hohen Drücken und dem toxischen Kohlenmonoxid, toxische oder teure Katalysatoren und die langwierige, nebenproduktreiche Umsetzung von Aminen mit Dimethylcarbonat zu vermeiden.

Die als Ausgangsstoffe II eingesetzten Oxalsäureesteramide können beispielsweise durch die Umsetzung von Aminen mit Oxalsäuredialkylester leicht hergestellt werden. Bevorzugte Ausgangsstoffe II und dementsprechend bevorzugte Endstoffe I sind solche, in deren Formeln $R^1$ einen Alkylrest mit 1 bis 18, insbesondere 1 bis 10 Kohlenstoffatomen, einen Cycloalkylrest mit 5 bis 8 Kohlenstoffatomen, einen Aralkylrest oder Alkylarylrest mit 7 bis 12 Kohlenstoffatomen oder einen, gegebenenfalls durch ein oder zwei Chloratome und/oder Alkoxygruppen mit 1 bis 4 Kohlenstoffatomen substituierten Phenylrest bedeutet. Die vorgennanten Reste können noch durch unter den Reaktionsbedingungen inerte Gruppen und/oder Atome, z.B. Alkylgruppen, Alkylmercaptogruppen, Alkoxygruppen, Alkyl- und Dialkylaminogruppen mit jeweils 1 bis 5 Kohlenstoffatomen je Alkylgruppe, Cyanogruppen, Nitrogruppen, Bromatome, Chloratome, substituiert sein.

Es kommen beispielsweise als Ausgangsstoffe II in Frage: Der Methyl-, Äthyl-, n-Propyl-, iso-Propyl-, n-Butyl-, sek.-Butyl-, tert.-Butyl-, 2-Methylbutyl-(1)-, 3-Methylbutyl-(1)-, 2-Methylbutyl-(2)-, 3-Methylbutyl-(2)-, Pentyl-(1)-, Pentyl-(2)-, Pentyl-(3)-, neo-Pentyl-, n-Hexyl-, n-Octyl-, Decyl-, Cyclopentyl-, Cyclohexyl-, Benzyl-, Phenylester des Oxalsäuremonomethylamids; entsprechende in 2-, 3- oder 4-Stellung einfach oder in 2,3-, 2,4-, 2,5-, 2,6-, 3,4-, 3,5-Stellung gleich oder unterschiedlich zweifach durch ein Chloratom, eine Methyl-, Äthyl-, Methoxy-, Äthoxygruppe substituierte Phenylester des Oxalsäuremonomethylamids; homologe durch die Äthyl-, n-Propyl-, iso-Propyl-, n-Butyl-, iso-Butyl-, sek.-Butyl-, 2-Methylbutyl-(1)-, 3-Methylbutyl-(1)-, 3-Methylbutyl-(2)-, Pentyl-(1)-, Pentyl-(2)-, Pentyl-(3)-, neo-Pentyl-, n-Hexyl-, n-Octyl-, Decyl-, Cyclopentyl-, Cyclohexyl-, Benzyl-, Phenylgruppe oder durch eine in 2-, 3- oder 4-Stellung einfach oder in 2,3-, 2,4-, 2,5-, 2,6-, 3,4-, 3,5-Stellung gleich oder unterschiedlich zweifach durch ein Chloratom, eine Methyl-, Äthyl-, Methoxy-, Äthoxygruppe substituierte Phenylgruppe substituiertes Monoamid vorgenannter Oxalsäuremonoester.

Die Umsetzung wird bei einer Temperatur von 300 bis 900°C, vorzugsweise von 400 bis 700°C, mit Unterdruck, drucklos oder unter Überdruck, vorzugsweise bei 0,001 bis 40, insbesondere 0,005 bis 1 bar, diskontinuierlich oder vorzugsweise kontinuierlich durchgeführt. Zweckmässig verwendet man als Reaktionsräume Reaktionsrohre mit Durchmessern von 20 bis 1000 Millimetern und Längen von 100 bis 2000 Millimetern mit Füllkörpern von 10 bis 50 Millimeter Durchmesser. Als Füllkörper können z.B. Raschigringe, Glasringe, Drahtnetzringe, Pallringe, Stahlwolle, Eisen-, Kupferspäne verwendet werden.

Zweckmässig verwendet man unter den Reaktionsbedingungen inerte Lösungsmittel. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; und entsprechende Gemische. Zweckmässig verwendet man das Lösungsmittel in einer Menge von 400 bis 10 000 Gewichtsprozent, bezogen auf Ausgangsstoff II.

Die Reaktion kann wie folgt durchgeführt werden: Der Ausgangsstoff in fester, flüssiger oder gasförmiger Form, gegebenenfalls im Gemisch mit Inertgas und/oder Lösungsmittel, wird durch den Reaktionsraum bei der Zersetzungstemperatur geleitet. Durchleiten eines Strippgases oder Anlegen von Vakuum bzw. Verwendung eines inerten Lösungsmittels ist vorteilhaft, um die Spaltprodukte rasch aus dem Spaltreaktor auszuführen und gegebenenfalls eine effektive Trennung von Endstoff und gebildetem Alkohol zu erleichtern. Zweckmässig richtet sich der in dem Reaktor einzustellende Druck normalerweise nach den Siedepunkten der gebildeten Reaktionsprodukte Isocyanat I und Alkohol. Man wird bevorzugt Drücke verwenden, die es erlauben, den Endstoff rasch aus dem Reaktor auszuführen, die aber eine fraktionierte Kondensation noch ermöglichen. Die Belastung beträgt zweckmässig 10 bis 500, insbesondere 50 bis 300 Gramm Ausgangsstoff II pro Liter Reaktionsraum und Stunde. Der Endstoff kann gegebenenfalls direkt verwendet oder in üblicher Weise, z.B. durch fraktionierte Destillation oder Kristallisation, gereinigt werden.

Die nach dem Verfahren der Erfindung herstellbaren Isocyanate sind wertvolle Ausgangsstoffe für die Herstellung von Pflanzenschutzmitteln, Schädlingsbekämpfungsmitteln, Farbstoffen, Kunstharzen und Kunststoffen, Textilhydrophobierungsmitteln, Waschmitteln und Klebstoffen. Bezüglich der Verwendung wird auf vorgenannte Veröffentlichungen und Ullmanns Encyklopädie der technischen Chemie, Band 9, Seiten 11, 12, 404, und Band 17, Seite 204, verwiesen.

Die in den folgendne Beispielen aufgeführten Teile bedeuten Gewichtsteile. Sie verhalten sich zu den Volumenteilen wie Kilogramm zu Liter.

Beispiel 1

20 Teile Oxalsäuremethylester-N-phenylamid werden innerhalb 45 Minuten über eine Dosierschnecke von oben in einen 160 Volumenteile fassenden Rohrreaktor eingebracht, der mit Drahtringen gefüllt ist und in dessen Inneren eine Temperatur von 600°C und ein Druck von 19 mbar herrschen. Das untere Ende des Reaktors mündet in eine Vorlage, die mit Wasser gekühlt wird; in ihr wird das gebildete Phenylisocyanat kondensiert. Eine zweite, mit Trockeneis gekühlte Vorlage zur Kondensation des Methanols ist nachgeschaltet.

Man erhält bei einem Umsatz von (gaschromatographisch bestimmt) 78 Prozent 9,5 Teile Phenylisocyanat (91,6% der Theorie) vom Kp. 164–67°C.

Beispiele 2 bis 7

Die weiteren Beispiele werden analog Beispiel 1 durchgeführt, wobei jedoch Ausgangsstoff, Temperatur, Zeit, Druck und Reaktorfüllung variiert werden. Die Ergebnisse dieser Umsetzungen sind in der Tabelle aufgeführt.

**Tabelle**

| Beisp. | Ausgangsprodukt | Isocyanat | Temp. °C | Druck mbar | Füllmaterial | Umsatz % | Ausbeute % der Theorie | Raumzeit ausbeute Volumenteile/l Reaktions-raum × Stunde |
|---|---|---|---|---|---|---|---|---|
| 2 | 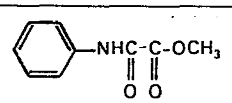 | 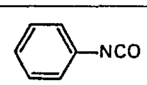 | 600 | 19 | Stahlwolle | 71 | 84 | 97 |
| 3 | 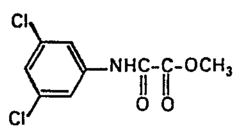 | 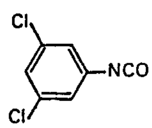 | 575 | 27 | Drahtnetzringe | 100 | 98 | 186 |
| 4 | 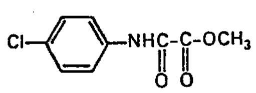 | 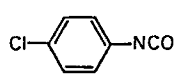 | 580 | 24 | Drahtnetzringe | 93 | 87 | 146 |
| 5 | 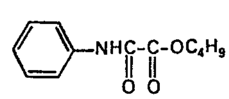 | 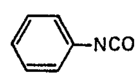 | 590 | 15 | Stahlwolle | 88 | 72 | 85 |
| 6 | 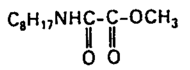 | $C_8H_{17}NCO$ | 575 | 29 | Drahtnetzringe | 64 | 44 | 51 |
| 7 | 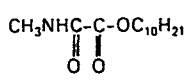 | $CH_3NCO$ | 600 | 40 | Drahtnetzringe | 57 | 88 | 29 |

Beispiel 8

Eine Lösung von 200 Teilen Oxalsäuremethylester-N-phenylamid in 400 Teilen o-Xylol wird innerhalb 190 Minuten in einen auf 280°C geheizten Verdampfer eingeführt. Die erhaltenen Dämpfe werden unter Verwendung von 3 Volumenteilen Stickstoff je Volumenteil Reaktionsgemisch und Stunde durch einen 160 Volumenteile fassenden Spaltreaktor geleitet, der mit Stahlwolle gefüllt ist und dessen Innentemperatur 590°C beträgt. Das gasförmige Reaktionsgemisch wird fraktioniert destilliert. Man erhält bei einem Umsatz von 86% (gaschromatographisch bestimmt) 93,8 Teile Phenylisocyanat (82% der Theorie) vom Kp. 54–55°C/17 mbar.

**Patentanspruch**

Verfahren zur Herstellung von Isocyanaten der Formel

$R^1\text{-}N{=}C{=}O$ I,

worin $R^1$ einen aliphatischen, cyloaliphatischen, araliphatischen, aliphatisch-aromatischen, aromatischen Rest bezeichnet, durch thermische Spaltung von Carbonamidoverbindungen, dadurch gekennzeichnet, dass man Oxalsäureesteramide der Formel

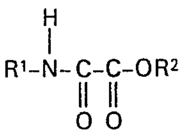

II,

worin $R^1$ die vorgenannte Bedeutung besitzt und $R^2$ einen aliphatischen, cycloaliphatischen, araliphatischen, aliphatisch-aromatischen oder aromatischen Rest bezeichnet, bei einer Temperatur von 300 bis 900°C thermisch spaltet.

**Claim**

A process for the preparation of an isocyanate of the formula

$R^1\text{-}N{=}C{=}O$ I,

where $R^1$ is an aliphatic, cycloaliphatic, araliphatic, aliphatic-aromatic or aromatic radical, by thermal cleavage of a carboxamido compound, wherein an oxalic acid ester amide of the formula

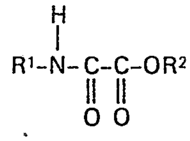

II,

where $R^1$ has the above meaning and $R^2$ is an aliphatic, cycloaliphatic, araliphatic, aliphatic-aromatic or aromatic radical, is cleaved thermally at a temperature of from 300 to 900°C.

**Revendication**

Procédé de préparation d'isocyanates de la formule

$R^1\text{-}N{=}C{=}O$ I,

dans laquelle $R^1$ désigne un groupe aliphatique, cycloaliphatique, araliphatique, aliphatique-aromatique ou aromatique, par décomposition thermique de composés carbamidiques, caractérisé en ce que l'on soumet à une décomposition thermique à une température comprise entre 300 et 900°C des amides d'esters de l'acide oxalique de la formule

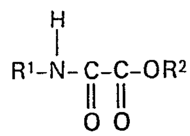

II,

dans laquelle $R^1$ possède l'une des significations définies ci-dessus et $R^2$ désigne un groupe aliphatique, cycloaliphatique, araliphatique, aliphatique-aromatique ou aromatique.